# EUROPEAN PATENT APPLICATION

(11) **EP 4 122 374 A1**
(43) Date of publication of application: **25.01.2023**
(21) Application number: 22185641.2
(22) Date of filing: 19.07.2022
(51) Int. Cl.: A61B 3/00, A61B 3/028, A61B 3/032

(54) **TARGET PRESENTATION DEVICE AND TARGET PRESENTATION PROGRAM**

(30) Priority: 20.07.2021 JP 2021119275; 20.07.2021 JP 2021119276
(71) Applicant: Nidek Co., Ltd., Aichi 443-0038 (JP)
(72) Inventor: SHIBATA, Kazunori, Gamagori, Aichi 4430038 (JP); HIRAYAMA, Yukito, Gamagori, Aichi 4430038 (JP); YAMAMOTO, Kunihiko, Gamagori, Aichi 4430038 (JP)
(74) Representative: Hoefer & Partner Patentanwälte mbB

(57) **Abstract**

A target presentation device for presenting a target to a subject eye. The target presentation device including a format setting means that sets a display format of a visual acuity value of an examination target used in an examination of the subject eye, based on an operation signal, and an output means that outputs a target to be presented to the subject eye, based on the visual acuity value represented in the display format set by the format setting means.

## Description

### TECHNICAL FIELD

The present disclosure relates to a target presentation device and a target presentation program for presenting an examination target to a subject eye.

### BACKGROUND ART

A target presentation device for presenting a target to a subject eye is known. For example, in JP-A-2008-023129, a target displayed on a display is switched by a target switching signal.

A first problem will be described. In inspecting a subject eye, different types of targets are presented with different visual acuity values. There are a plurality of display formats for the visual acuity values of the targets. For example, display formats vary depending on countries or regions. However, according to a device in the related art, a display format of the visual acuity value is fixed in advance, and the display format is not desired by an examiner.

A second problem will be described. In inspecting the subject eye, an alignment of the targets is patterned in advance so that contents of the targets displayed on a display do not overlap each other. However, since diversified examinations are carried out in recent years, the inventors have studied a technique for displaying contents of the targets in a free combination. In this case, there is a possibility that the alignment of the targets is not changed even when the targets are switched, thereby affecting an examination result.

### SUMMARY OF INVENTION

An object of the present disclosure is to provide a target presentation device and a target presentation program which can improve convenience of an examiner. In addition, another object of the present disclosure is to provide a target presentation device and a target presentation program which can accurately inspect a subject eye.

In order to achieve the above-described objects, the present disclosure includes the following configurations.

A target presentation device for presenting a target to a subject eye, the target presentation device including:
a format setting means that sets a display format of a visual acuity value of an examination target used in an examination of the subject eye, based on an operation signal; and
an output means that outputs a target to be presented to the subject eye, based on the visual acuity value represented in the display format set by the format setting means.

A target presentation program used in a target presentation device for presenting a target to a subject eye,
in which the target presentation program includes instructions which, when the program is executed by a processor of the target presentation device, cause the target presentation device to perform:
a format setting step of setting a display format of a visual acuity value of an examination target used in an examination of the subject eye, based on an operation signal; and
an output step of outputting a target to be presented to the subject eye, based on the visual acuity value represented in the display format set in the format setting step.

### BRIEF DESCRIPTION OF DRAWINGS

FIG. 1 is an external view of a target presentation device.
FIG. 2 is a schematic view of a control system of the target presentation device.
FIG. 3 illustrates examples of a target displayed on an LCD.
FIG. 4 illustrates examples of a setting screen.
FIG. 5 is a view illustrating a step of the target.
FIG. 6 illustrates an example of an examination screen.
FIG. 7 illustrates examples of a current horizontal single-row target and a subsequent horizontal single-row target.
FIG. 8 illustrates a modification example of the target.

### DESCRIPTION OF EMBODIMENTS

### <Overview>

An embodiment of a target presentation device according to the present disclosure will be described. Items classified by <> below may be used independently or in association with each other. In the present embodiment, an examination target used in an examination of a subject eye will be referred to as an "examination target". The examination target may be a single target having a predetermined visual acuity value, or may include a plurality of targets having different visual acuity values.

The target presentation device of the present embodiment is a device for presenting the target to the subject eye. For example, as the target, a target having an orientation for an examinee to identify directions (as an example, a Landolt ring target or a tumbling E target), and a character target for the examinee to read characters (as an example, a hiragana target, a katakana target, or an alphabet target), and a numerical symbol target for the examinee to identify numerical symbols may be output. In addition, for example, as the target, a target including a single target (for example, a single character target) and a target group including a plurality of targets (as an example, a horizontal single-row target, a vertical single-row target, or a character crowding target) may be output.

### <Format Setting Means>

The target presentation device of the present embodiment may include a format setting means (for example, a control unit 20). The format setting means sets a display format of a visual acuity value of an examination target used in the examination of the subject eye, based on an operation signal (first operation signal). In this manner, an examiner can confirm the visual acuity value of the target by a desired display format, and thus, convenience of the target presentation device can be improved.

The display format of the visual acuity value of the examination target is a display format represented based on a visual angle of the target, a size of the target (size corresponding to the visual acuity value), an examination distance, and the like. The visual angle of the target can be considered by replacing the visual angle with the size of the target. For example, the display format based on the visual angle of the target may be a decimal display, and the display format based on the examination distance may be a fractional display. As a matter of course, a display format different from the decimal display and the fractional display may be used.

The decimal display indicates that a predetermined visual angle of the target can be identified. For example, in a case where the visual angle of one minute of the target can be identified, the visual acuity value is represented as 1.0. For example, in a case where the visual angle of 0.5 minutes of the target can be identified, the visual acuity value is represented as 2.0. The decimal display is frequently used in Japan.

As an example, in a case where the Landolt ring target is used as an example, when the examination distance is 5 m, a cut and a line width of the Landolt ring target is set to approximately 1.45 mm, and a size of an outer diameter of the Landolt ring target is set to approximately 7.27 mm. In this manner, the cut and the line show the visual angle of one minute, and show the Landolt ring target with the visual acuity value of 1.0. In addition, the cut and the line width of the Landolt ring target are set to approximately 0.73 mm, and the size of the outer diameter of the Landolt ring target is set to approximately 3.64 mm. In this manner, the cut and the line show the visual angle of 0.5 minutes, and show the Landolt ring target with the visual acuity value of 2.0.

The fractional display indicates that a predetermined visual angle of the target can be identified at a predetermined examination distance. For example, a distance at which an eye (reference eye) that can visually recognize the visual angle of one minute of the target can identify the target is represented as a denominator. For example, the examination distance of the target with respect to the subject eye is represented as a molecule. This fractional display is frequently used overseas.

As an example, in a case where the target which can be identified by the reference eye (visual acuity value of 20/20) at 20 feet can be visually recognized by the subject eye at the examination distance of 20 feet, the visual acuity value is represented as 20/20. In a case where the target which can be identified by the reference eye at 10 feet can be visually recognized by the subject eye at the examination distance of 20 feet, the visual acuity value is represented as 20/10. The examination distance of 20 feet is a distance mainly used in the United States. In addition, as an example, in a case where the target which can be identified by the reference eye (visual acuity value of 6/6) at 6 m can be visually recognized by the subject eye at the examination distance of 6 m, the visual acuity value is represented as 6/6. In a case where the target which can be identified by the reference eye at 3 m can be visually recognized by the subject eye at the examination distance of 6 m, the visual acuity value is represented as 6/3. The examination distance of 6 m is a distance mainly used in the United Kingdom.

The visual angle of the target (size of the target) and the examination distance are equivalent, and the decimal display can be replaced with the fractional display. For example, the visual acuity value of 2.0 corresponds to a fact that the target having the visual angle of one minute at the examination distance of 5 m can be identified by the subject eye at the examination distance of 10 m. Therefore, it is considered that the target which can be identified by the reference eye (visual acuity value of 1.0) at 5 m can be visually recognized by the subject eye at the examination distance of 10 m. Accordingly, the display can be replaced with the visual acuity value of 10/5, the fractional display. The visual acuity value 20/10 and the visual acuity value 6/3 can be replaced with the visual acuity value 2.0, the decimal display, by performing a decimal calculation.

In the present embodiment, the operation signal for setting the display format of the visual acuity value of the examination target may be output by the examiner operating an operation means. For example, the operation means may directly select the display format of the visual acuity value of the examination target. In this case, the format setting means may set the display format of the visual acuity value of the examination target, based on the operation signal indicating the decimal display or the fractional display from the operation means. In addition, for example, the operation means may select a country or a region to designate the display format of the visual acuity value of the examination target. In this case, the country or the region may be associated with the decimal display or the fractional display in advance. The format setting means may set the display format of the visual acuity value of the examination target, based on the operation signal indicating the country or the region from the operation means. With regard to the fractional display, the examination distance may be set. For example, at least one of 20 feet and 6 m may be set.

In addition, in the present embodiment, the operation signal for setting the display format of the visual acuity value of the examination target may be output by using an external storage means (for example, an SD card, a USB memory, a server, or a cloud). In this case, a control means included in a device main body or the operation means may read the setting of the display format of the visual acuity value of the examination target stored in the external storage means, and may output the operation signal corresponding to the setting. The format setting means may set the display format of the visual acuity value of the examination target, based on the operation signal described above.

### <Step Setting Means>

The target presentation device of the present embodiment may include a step setting means (for example, a control unit 20). The step setting means sets the visual acuity values of a plurality of targets included in the examination target to have a predetermined step, based on the display format of the visual acuity value of the examination target set by the format setting means. In this manner, a visual acuity value group which can be presented in the examination of the subject eye is easily set.

The predetermined step indicates a variation between the visual acuity values (in other words, a variation between the visual acuity value of the target and the visual acuity value of previous or next target) in a case where the visual acuity values of the plurality of targets are aligned in order. A change in the visual acuity values of the plurality of targets can also be represented as a change in the visual angles based on the examination distance. In this case, the predetermined step may indicate a variation between the visual angles in a case where the visual angles of the plurality of targets are aligned in order.

More specifically, the predetermined step is a step in which the visual acuity values of the plurality of targets regularly increase. For example, the predetermined step may be a decimal step in which at least a part of the visual acuity values of the plurality of targets linearly increases. In this case, the visual acuity values of the plurality of targets have equal differences in decimal units. In addition, for example, the predetermined step may be a logarithmic step in which at least a portion of the visual acuity values of the plurality of targets logarithmically increases. In this case, the visual acuity values of the plurality of targets have equal intervals in units of exponents (or power exponents).

The visual acuity values of the plurality of targets may include values which are not included in the predetermined step. For example, the decimal step may include values which does not have equal differences, even based on a linear increase in the visual acuity value. Therefore, the step setting means may set the decimal step in more detail. Similarly, for example, the logarithmic step may include values which does not have equal intervals, even based on a logarithmic increase in the visual acuity value. Therefore, the step setting means may set the logarithmic step in more detail. As an example, a 20-foot step in which the visual acuity value of 20/70 is added to the logarithmic step or a 6-m step in which the visual acuity value of 6/20 is added to the logarithmic step may be set.

In the present embodiment, the display format of the visual acuity value of the examination target and the predetermined step may be associated with each other in advance. As an example, the decimal display may be associated with the decimal step, and the fractional display may be associated with the logarithmic step. As a matter of course, a 20-foot step and a 6-m step may be associated with the examination distance in the fractional display. In this case, the step setting means may set the display format of the visual acuity value of the examination target and the predetermined step corresponding thereto, based on the operation signal (first operation signal) indicating the fractional display or the decimal display.

### <Step Changing Means>

The target presentation device of the present embodiment may include a step changing means (for example, the control unit 20). The step changing means changes the predetermined step set by the step setting means based on the display format of the visual acuity value, to a step different from the predetermined step based on an operation signal (second operation signal). In other words, the step changing means changes an initial setting step to a different step. In this manner, the visual acuity value group based on a change in the visual acuity values of the plurality of targets can be changed when needed.

In the present embodiment, the operation signal for changing the step of the visual acuity values of the plurality of targets included in the examination target may be output by the examiner operating the operation means. In this case, the step changing means may change the initial setting step to the different step, based on the operation signal indicating a step change from the operation means. For example, an initial-set decimal step may be changed to the logarithmic step, or the initial-set logarithmic step may be changed to the decimal step. As a matter of course, a change from the decimal step to the 20-foot step, or a change from the 20-foot step to the 6-m step may be included.

In addition, in the present embodiment, the operation signal for changing the step in the visual acuity values of the plurality of targets included in the examination target may be output by using the external storage means. The step changing means may change the visual acuity value of the examination target to a different step, based on the operation signal transmitted via the external storage means.

### <Step Selection Means>

The target presentation device of the present embodiment may include a step selection means (for example, the control unit 20). The step selection means sets the change in the visual acuity values of the plurality of targets which can be represented in the display format of the visual acuity value of the examination target, to any step, based on an operation signal (third operation signal). For example, even in a case where the predetermined step is not associated with the display format of the visual acuity value of the examination target, either the decimal step or the logarithmic step can be set. In addition, with regard to the logarithmic step, either the 20-foot step or the 6-m step may be set. In this manner, the visual acuity value group based on the change in the visual acuity values of the plurality of targets can be set in accordance with a purpose or content of the examination of the subject eye.

In the present embodiment, the operation signal for selecting the step of the visual acuity values of the plurality of targets included in the examination target may be output by the examiner operating the operation means. In this case, the step selection means may set any desired step, based on the operation signal designating the step from the operation means.

In addition, in the present embodiment, the operation signal for selecting the step of the visual acuity values of the plurality of targets included in the examination target may be output by using the external storage means. The step selection means may set the visual acuity value of the examination target to any desired step, based on the operation signal transmitted via the external storage means.

### <Target Adding Means>

The target presentation device of the present embodiment may include a target adding means (for example, the control unit 20). The target adding means adds the target having a new visual acuity value represented in the display format of the visual acuity value of the examination target, based on an operation signal (fourth operation signal). For example, a new visual acuity value group may be formed by adding the visual acuity value from the beginning without setting the predetermined step or any desired step for the visual acuity values of the plurality of targets. In addition, for example, the existing visual acuity value group may be customized by appropriately adding the visual acuity value which is not included in the predetermined step or any desired step to the visual acuity values of the plurality of targets.

In the present embodiment, the operation signal for adding the new visual acuity value may be output by the examiner operating the operation means. In this case, the target adding means may add the target having the new visual acuity value, based on the operation signal from the operation means.

In addition, in the present embodiment, the operation signal for adding the new visual acuity value may be output by using the external storage means. The target adding means may add the target having the new visual acuity value, based on the operation signal transmitted via the external storage means.

### <Type Setting Means>

The target presentation device of the present embodiment may include a type setting means (for example, the control unit 20). The type setting means sets a type of the examination target, based on an operation signal (fifth operation signal). More specifically, the type of the examination target which can be presented in the examination of the subject eye is set. For example, displaying and non-displaying of the Landolt ring target, the tumbling E target, the hiragana target, the katakana target, the alphabet target, or the numerical symbol target described above may be set.

In the present embodiment, the operation signal for setting the type of the examination target may be output by the examiner operating the operation means. For example, the operation means may directly select the type of examination target. In this case, the type setting means may set the type of the examination target, based on the operation signal indicating displaying or non-displaying of each type output from the operation means. The country or the region may be associated with the type of the examination target used in the examination in advance, the operation signal may be acquired from the selection of the country or the region, and the type of examination target may be set. In addition, the type of the examination target may be set in conjunction with the setting of the fractional display or the decimal display, based on the operation signal when the format setting means sets the display format of the visual acuity value of the examination target. That is, the first operation signal may also serve as the fifth operation signal.

In addition, in the present embodiment, the operation signal for setting the type of the examination target may be output by using the external storage means. The type setting means may set the type of the examination target, based on the operation signal transmitted via the external storage means.

### <Signal Acquisition Means>

The target presentation device of the present embodiment may include a signal acquisition means (for example, the control unit 20). The signal acquisition means acquires a switching signal for switching a first target group that is described later to be displayed by target presenting means. For example, the signal acquisition means may acquire the switching signal output by the examiner operating the operation means. As an example, the switching signal may be acquired by using, as a trigger, an operation for switching the types of the target, an operation for switching the visual acuity values of the target, an operation for switching the displays of the target, or the like. In a case where the target has an orientation, the switching signal may be acquired by using, as trigger, an operation for switching the directions of the target.

In addition, for example, the signal acquisition means may acquire the switching signal, based on a self-examination program which automatically performs the examination on the subject eye. In this case, the switching signal output by the examiner operating the operation means for the examiner may be acquired. In addition, in this case, a switching signal that is automatically output in a case where a predetermined time elapses from a presentation timing of the target, may be acquired.

### <Generation Means>

The target presentation device of the present embodiment may include a generation means (for example, the control unit 20). The generation means randomly aligns the plurality of targets in a straight line in at least one of a column direction and a row direction to generate a first target group displayed on an identical screen of the target display means. In addition, the generation means randomly aligns the plurality of targets in a straight line in at least one of the column direction and the row direction to generate a second target group displayed on the identical screen of the target display means, based on the switching signal from the signal acquisition means. For example, as the first target group or the second target group, the generation means may generate any one of the horizontal single-row target in which the plurality of targets are aligned in the row direction, the vertical single-row target in which the plurality of targets are disposed in the column direction, and the character crowding target in which the plurality of targets are aligned in the column direction and the row direction. In the first target group and the second target group, at least one of the number of the plurality of targets forming the first target group and the second target group, a distance between the plurality of targets forming the first target group and the second target group, and a display position of the plurality of targets forming the first target group and the second target group, can be determined by the examiner.

The first target group may include the plurality of targets having an identical first visual acuity value. That is, the vertical single-row target and the horizontal single-row target may include the plurality of targets having the first visual acuity value. The character crowding target may include at least one row of the plurality of targets having the first visual acuity value.

In addition, the second target group may include at least one of the plurality of targets having the identical first visual acuity value and the plurality of targets having a second visual acuity value different from the identical first visual acuity value. That is, the vertical single-row target and the horizontal single-row target may include the plurality of targets having the first visual acuity value or the plurality of targets having the second visual acuity value. The character crowding target may include at least one row of the plurality of targets having the first visual acuity value, or at least one row of the plurality of targets having the second visual acuity value. As a matter of course, the character crowding target may serve as the target in which the plurality of targets having the first visual acuity value and the plurality of targets having the second visual acuity value are combined with each other.

In the present embodiment, in a case where the generation means randomly disposes the plurality of targets, the generation means sets the targets adjacent to each other to have different contents. For example, the generation means may set the targets, included in the first target group and the second target group, adjacent to each other to have different directions, different characters, and different numerical symbols. That is, in the first target group and the second target group, the plurality of targets are aligned so that the same direction, the same character, and the same numerical symbol are not continuous in the column direction and the row direction. In this manner, even in a case where an alignment of the target groups is not stored in advance, the targets unsuitable for the examination of the subject eye is not arranged, and accuracy of the examination of the subject eye can be improved.

In a case where the targets forming the first target group and the second target group have orientations, the generation means may randomly align the targets so that directions of the targets do not overlap each other at least in the row direction. As a matter of course, in a case where the number of the targets forming each target group is larger than the number of the directions of the targets, the directions may overlap each other. In addition, in a case where the targets forming the first target group and the second target group have orientations, the generation means may randomly dispose the targets so that the vertical direction and the horizontal direction of the targets are not biased at least in the row direction. In this manner, the examination considering astigmatism of the subject eye can be proceeded.

In the present embodiment, in a case where an order of alignment of the plurality of targets forming the second target group (in other words, alignment of the plurality of targets forming the second target group in the column direction and the row direction) is identical to an order of alignment of the plurality of targets forming the first target group, and a display position of the target forming the second target group overlaps a display position of the target forming the first target group, the generation means may set the targets to have different contents. Overlapping of the display positions referred to here does not mean that the display position is completely identical pixel position in the target display means. For example, in the first target group and the second target group, the pixel position may be shifted to such an extent that there is no significant difference in the display positions of the individual targets visually recognized by the subject eye. In this manner, the examinee is less likely to remember the targets, and accuracy of the examination of the subject eye can be improved.

In a case where the order of the alignment of the plurality of targets forming the second target group is different from the order of the alignment of the plurality of targets forming the first target group, the targets may be set to have identical content or may be set to have different contents when the display positions of the targets overlap each other.

### <Output Means>

The target presentation device of the present embodiment may include an output means. The output means outputs the target to be presented to the subject eye, based on the visual acuity value represented in the display format set by the format setting means. For example, the output means may be a target display means (for example, LCD 3) for displaying the target. In this case, the output means (target display means) may display the target having a size corresponding to the visual acuity value. In addition, for example, the output means may be a printing means (for example, a printer) for printing the target. In this case, the output means (printing means) may print the target having a size corresponding to the visual acuity value. In addition, for example, the output means may be a storage means for storing the target. The storage means may be a storage means inside the device (for example, a memory), or may be a storage means outside the device. In this case, the output means (storage means) may store the target having a size corresponding to the visual acuity value. As a matter of course, the output means may be a means different from the above-described means.

### <Display Control Means>

The target presentation device of the present embodiment may include a display control means (for example, the control unit 20). The display control means controls the target display means, and causes the target display means to display the target. The target may be the target group generated by the generation means. The first target group may be displayed on the target display means, or the second target group may be displayed on the target display means. In this case, the screen display of the target display means may be switched from the first target group to the second 8 8 target group.

The display control means may acquire a display size for displaying the target on the target display means, based on the visual acuity value input from a visual acuity value setting screen corresponding to the display format of the visual acuity value of the examination target. In this case, the display size of the target displayed on the target presenting means may be acquired by using correspondence information (as an example, at least one of a correspondence table, a calculation expression, and the like) in which the visual acuity value and the display size are associated with each other.

The visual acuity value setting screen corresponding to the display format of the visual acuity value of the examination target may be a screen for setting the visual acuity value, and may be a screen switched to display each display format. As an example, in a case where the decimal display is set, the decimal step associated with the decimal display may be applied, and the visual acuity value setting screen including the visual acuity value group based on the decimal step may be displayed. As an example, in a case where the logarithmic display is set, the decimal step associated with the logarithmic display may be applied, and the visual acuity value setting screen including the visual acuity value group based on the logarithmic step may be displayed. As a matter of course, the visual acuity value setting screen may be a screen different from the above-described screens. For example, the visual acuity value setting screen for each display format of the visual acuity value of the examination target can be switched in this way so that various settings can be easily performed or changed.

### <Operation Means>

The target presentation device of the present embodiment may include an operation means (for example, a controller 4). The operation means may be configured in any desired manner as long as the examiner can input the operation signal, and at least one user interface of as a mouse, a keyboard, a touch panel, a controller, and the like can be used. In a case where the operation means is the touch panel, the operation means also serves as a display means.

For example, the operation means can input the operation signal for operating the display of the target display means (for example, LCD 3). As an example, the operation signal for changing at least one of the type of the examination target, the visual acuity value of the examination target, the display of the examination target, and the like can be input. In addition, for example, the operation means can input the operation signal for operating the display of the display means (for example, LCD 41). As an example, the operation signal for changing at least one of the display format of the visual acuity value of the examination target, various setting screens, and the like can be input.

As a matter of course, the operation means may input the operation signal for an operating means different from the target display means and the display means. For example, the operation signal for operating an optical element in a calibration optical system (calibration means) that is described later may be input.

The target presentation device of the present embodiment may be used as a part of a visual acuity examination device for measuring the visual acuity by the examinee looking into a presentation window. That is, the target presentation device may be used as a target presentation means in the visual acuity examination device.

The target presentation device of the present embodiment may be used as a part of a subjective optometry device for subjectively measuring optical characteristic of the subject eye. That is, the target presentation device may be used as a target presenting means in the subjective optometry device. In this case, a target light flux from the target presentation device may be directly projected toward the subject eye. In addition, in this case, the target light flux may be projected via a projection optical system having at least one optical member (for example, a lens, a mirror, and the like) for allowing the target light flux from the target presentation device to pass therethrough.

In addition, the subjective optometry device may include a calibration optical system changing an optical characteristic of the target light flux from the target presentation device and disposed in an optical path of the projection optical system. In this case, the calibration optical system may change the optical characteristic of the target light flux by controlling an optical element disposed between the target presentation device and the optical member included in the projection optical system. That is, the calibration optical system may be a phantom lens refractometer (phantom calibration optical system). In addition, the calibration optical system may change the optical characteristic of the target light flux by controlling the optical element disposed in front of the subject eye. That is, the calibration optical system may be an optometry unit (phoropter). For example, the optometry unit may have a lens disk in which a plurality of optical elements are disposed on the same circumference and a driving means (for example, a motor) for rotating the lens disk, and may be configured to electrically switch the optical element by driving the driving means.

The present disclosure is not limited to the device described in the present embodiment. For example, terminal control software (program) that performs functions of the following embodiments can be supplied to a system or a device via a network or various storage media, and can be executed in such a way that a control device (for example, a CPU) of the system or the device reads a program.

### <Example>

An example of the target presentation device according to the present example will be described.

### <Device Configuration>

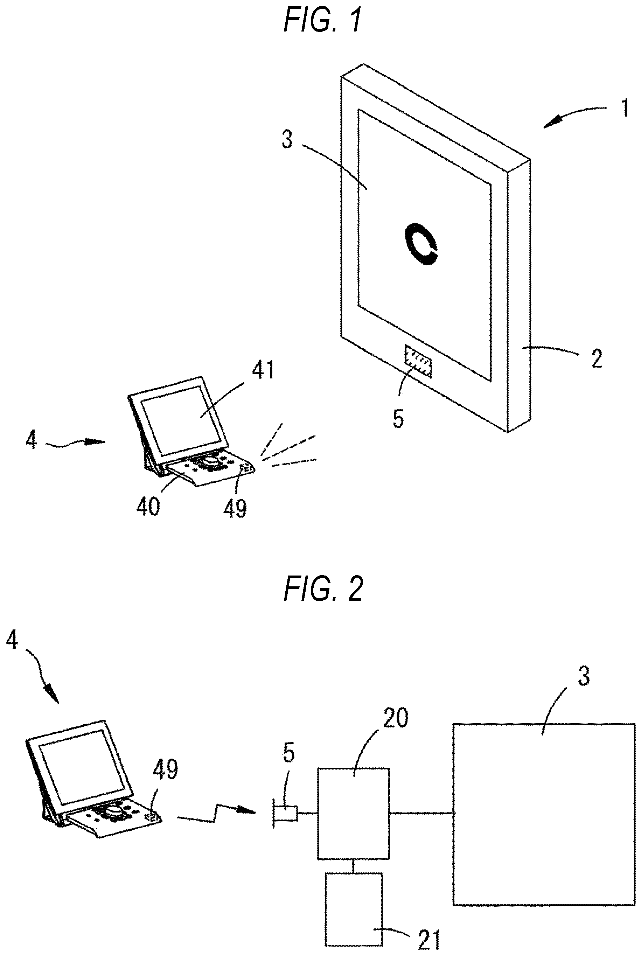

FIG. 1 is an external view of the target presentation device 1. A liquid crystal display (LCD) 3 for presenting a target is disposed on a front surface (forward surface) of a housing 2 in the target presentation device 1. The LCD 3 has a display screen having a size which can display the target having a visual acuity value of at least 0.1 to 2.0 or a size corresponding to this visual acuity value, even in a case where the LCD 3 is placed at a distant examination distance (for example, 5 m). A receiving unit 5 for receiving an operation signal transmitted from a controller 4 is disposed below the front surface of the housing 2. The targets displayed on the LCD 3 can be switched by an operation of the controller 4. The controller 4 includes a plurality of switch units 40 for operating a device main body, a liquid crystal display (LCD) 41 for displaying an operation status of each switch, and a transmitting unit 49 for transmitting an operation signal based on the operation of each switch. The LCD 41 may be a touch panel which also functions as the switch unit 40. In addition, the housing 2 and the controller 4 may be separately provided, and various operation signals may be transmitted by remote control.

FIG. 2 is a schematic view of a control system of the target presentation device 1. A control unit 20 is stored inside the housing 2. For example, the control unit 20 includes a CPU (processor), a RAM, and a ROM. The CPU controls driving of each part in the target presentation device 1. Various types of information are temporarily stored in the RAM Various programs executed by the CPU, a decoder circuit for decoding an operation signal from the controller 4, a reference image (vector image) of each target, and the like are stored in the ROM. The control unit 20 may include a plurality of control units (that is, a plurality of processors).

The LCD 3, the receiving unit 5, a non-volatile memory 21 (hereinafter, a memory 21), and the like are connected to the control unit 20. The receiving unit 5 provided in the housing 2 and the transmitting unit 49 provided in the controller 4 are connected by wireless communication. For example, the memory 21 is a non-transitory storage medium that can hold stored contents even in a case where power supply is cut off. For example, a hard disk drive, a flash ROM, a USB memory, or the like can be used as the memory 21.

### <Type of Target and Format of Visual Acuity Value>

There are various types of the targets that is presented to the subject eye. For example, the types include a target having an orientation for an examinee to identify directions (as an example, a Landolt ring target or a tumbling E target), and a character target for the examinee to read characters (as an example, a hiragana target, a katakana target, or an alphabet target), and a numerical symbol target for the examinee to identify numerical symbols.

The visual acuity value of the target is represented in various ways. In the present example, since the display format of the visual acuity value of the target can be set in any format, the visual acuity value can be represented in the display format desired by the examiner. Therefore, the examination can be efficiently promoted. Details of setting the display format will be described later.

### <Display of Target>

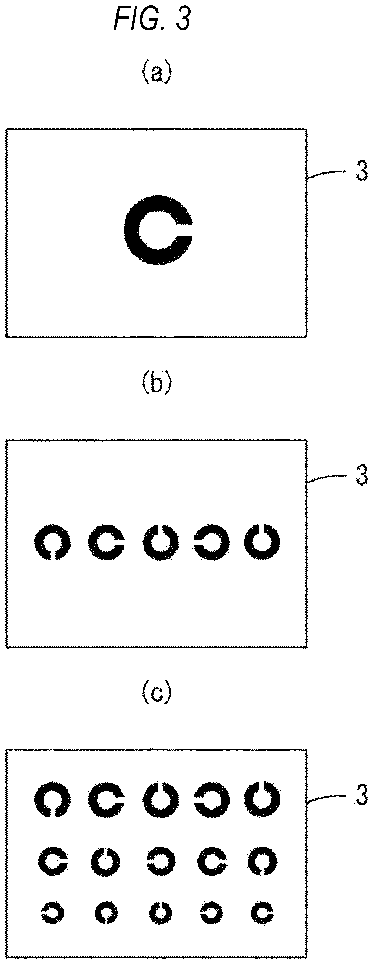

FIG. 3 illustrates examples of the target displayed on the LCD 3, and an example (a) of FIG. 3 illustrates a single-character target, an example (b) of FIG. 3 illustrates a horizontal single-row target, and an example (c) of FIG. 3 illustrates a character crowding target. In the present example, the Landolt ring target will be described as an example, but the same applies to other types of the targets.

The single-character target shown in the example (a) of FIG. 3 is the target having a single-Landolt ring target. For example, in the single-character target, the Landolt ring target is displayed at a middle stage position of the LCD 3. As a matter of course, the Landolt ring target may be displayed at a position different from a middle stage of the LCD 3.

The horizontal single-row target shown in the example (b) of FIG. 3 is a target group having a plurality of the Landolt ring targets. In the horizontal single-row target, the Landolt ring targets are disposed in a horizontal single row. Here, five Landolt ring targets are disposed in the horizontal single row (in other words, alignment of one row and five columns). For example, in the horizontal single-row target, a row of the Landolt ring target is displayed at the middle stage position of the LCD 3. As a matter of course, the row of the Landolt ring target may be displayed at a position different from the middle stage of the LCD 3.

The character crowding target shown in the example (c) of FIG. 3 is a target group having the plurality of Landolt ring targets. In the character crowding target, the Landolt ring targets are aligned in a plurality of column directions and a plurality of row directions. Here, 15 Landolt ring targets are aligned in 3 rows and 5 columns. The visual acuity values of the targets in each row may be identical to or different from each other. That is, the visual acuity values of the targets may be identical in all three rows, or the visual acuity values of the targets may be different in at least two rows among a first row, a second row, and a third row. For example, in the character crowding target, each row of the Landolt ring targets is displayed at an upper stage position, the middle stage position, and a lower stage position of the LCD 3. As a matter of course, each row of the Landolt ring targets may be disposed at a position different from that illustrated in the example (c) of FIG. 3.

In addition, in the horizontal single-row target and the character crowding target of the present example, at least one of the number of rows and the number of columns of the Landolt ring targets may be different from the above-described number. In addition, in a case where there are four or more Landolt ring targets aligned in a single row, a configuration in which all cut directions appear, namely an upward direction, a downward direction, a leftward direction, and a rightward direction, may be applied. Similarly, in a case where there are four or more Landolt ring targets aligned in a single row, a configuration in which all cut directions appear may be applied. Furthermore, the cut directions of the Landolt ring targets aligned in the single row may be set so that at least the targets adjacent to each other have different directions. Similarly, the cut directions in the Landolt ring targets aligned in the single row may also be set so that at least the targets adjacent to each other have different directions. That is, a configuration in which identical cut directions of the Landolt ring targets are not continuous in each of the column direction and the row direction, may be applied.

In the present example, each time the display screen of the LCD 3 is switched in the target presentation device 1, the cut direction of the Landolt ring target is randomly determined in accordance with the display of each target. The horizontal single-row target or the character crowding target is generated by randomly aligning the plurality of Landolt ring targets in a straight line in at least one of the column direction and the row direction. Details of the generation of the targets will be described later.

### <Control Action>

A control action of the target presentation device 1 will be described in an example where a visual acuity examination is performed on the subject eye by using the target presentation device 1.

### <Setting of Examination Target>

First, with regard to the examination target used in the examination of the subject eye, the examiner sets, in advance, a type of the examination target to be presented to the subject eye, a display format of the visual acuity value of the examination target, and the like. The examiner operates the switch unit 40 of the controller 4, and calls up a setting screen for setting the examination target. The control unit 20 causes the LCD 41 to display the setting screen in response to an operation signal from the switch unit 40.

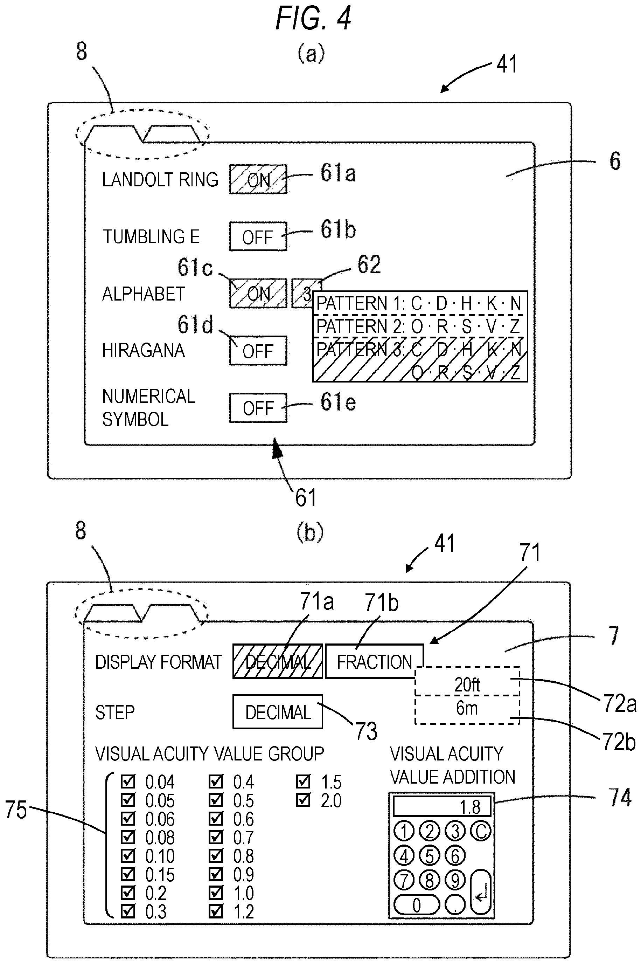

FIG. 4 illustrates examples of the setting screen, an example (a) of FIG. 4 illustrates a type setting screen 6 for setting the type of the examination target, and an example (b) of FIG. 4 illustrates a visual acuity value display setting screen 7 for setting the display format of the visual acuity value of the examination target. For example, the type setting screen 6 and the visual acuity value setting screen 7 can be switched by a tab 8. As a matter of course, for example, the type setting screen 6 and the visual acuity value setting screen 7 may be displayed as one integrated screen.

First, the examiner operates the type setting screen 6. The type setting screen 6 includes a display change button 61 for changing displaying and non-displaying on the LCD 3 for each type of examination target. For example, the display change button 61 includes a display change button 61a of the Landolt ring target, a display change button 61b of the tumbling E target, a display change button 61c of the alphabet target, a display change button 61d of the hiragana target, and a display change button 61e of the numerical symbol target.

The examiner operates the LCD 41 to select each display change button and switches between displaying and non-displaying for each examination target. For example, in the present example, the Landolt ring target and the alphabet target are changed to displaying, and the other targets are changed to non-displaying. The operation signal indicating displaying or non-displaying based on the operation of the LCD 41 is transmitted from the transmitting unit 49 to the receiving unit 5.

A fixed pattern in which a plurality of characters or a plurality of numerical symbols are grouped and associated with each other in advance may be provided, regarding the hiragana target, the katakana target, and the alphabet target in which a large number of character combinations can be generated, and the numerical symbol target in which a large number of numerical symbol combinations can be generated. In a case where the examiner switches the examination targets to displaying, a selection button 62 for selecting the fixed pattern appears. In addition, in a case where the examiner selects any fixed pattern from the selection button 62, an operation signal for designating the desired fixed pattern is transmitted from the transmitting unit 49. As a matter of course, in the hiragana target, the katakana target, the alphabet target, and the numerical symbol target, displaying and non-displaying may be switched by one character.

The receiving unit 5 receives the operation signal transmitted from the transmitting unit 49, and inputs the operation signal to the control unit 20. The control unit 20 sets the examination target which can be displayed on the LCD 3, based on at least one of the operation signal for designating displaying or non-displaying of the type of the examination target and the operation signal for designating the fixed pattern of the examination target.

Subsequently, the examiner operates the visual acuity value display setting screen 7. The visual acuity value display setting screen 7 includes a format change button 71 for setting the display format of the visual acuity value of the examination target. For example, the format change button 71 includes a decimal button 71a for representing the visual acuity value as a decimal number and a fractional button 71b for representing the visual acuity value as a fractional number.

The examiner operates the LCD 41 to select either the decimal button 71a or the fractional button 71b, and switches the display of the visual acuity value to the decimal display or the fractional display. Based on the operation signal from the LCD 41, the control unit 20 changes the display of the visual acuity value in the setting screen displayed on the LCD 41 and an examination screen 9 that is described later to the decimal display or the fractional display. For example, in the present example, the display format of the visual acuity value is changed to the decimal display by selecting the decimal button 71a.

In a case where the fractional button 71b is selected, a detail button for setting a more detailed display format may appear. As an example, a 20-foot button 72a for representing the visual acuity value at the examination distance of 20 feet, and a 6-m button 72b for representing the visual acuity value at the examination distance of 6 m may appear. As a matter of course, buttons having different display formats may appear. In a case where the examiner selects any display format from the detail button, the control unit 20 changes the display of the visual acuity value to a display at 20 feet or a display at 6 m.

Here, the examination target used in the examination of the subject eye includes a plurality of targets, and the plurality of targets have different visual acuity values. In other words, the examination target includes a visual acuity value group including different visual acuity values. For example, in the examination target, the visual acuity values of the plurality of targets are changed in any step of the decimal step and the logarithmic step.

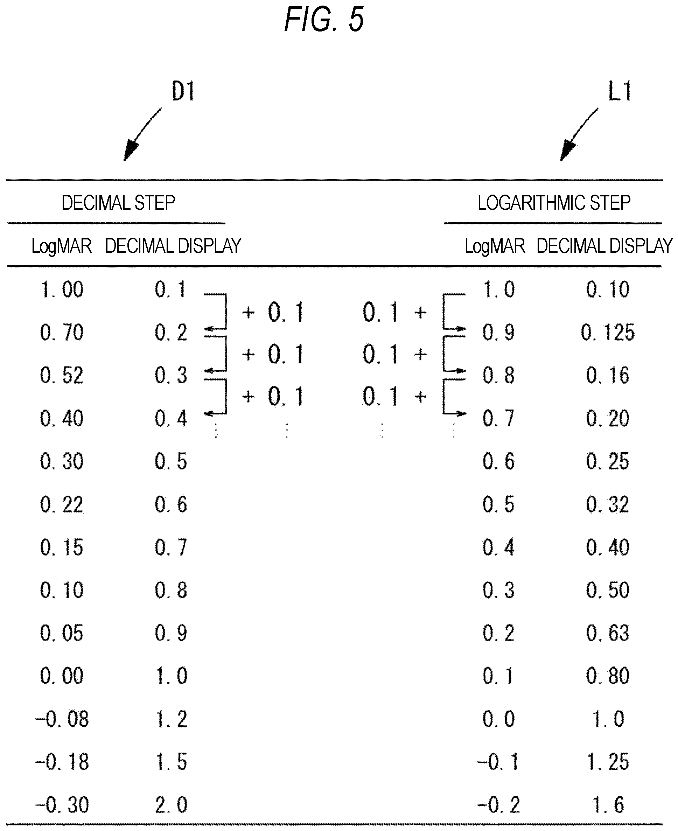

FIG. 5 is a view illustrating steps of the target. In FIG. 5, a case where the display format of the visual acuity value of the target is set to the decimal display is illustrated as an example. A decimal step D1 is a step in which variations in the visual acuity values of the targets are provided at an equal interval in decimal units. In other words, the decimal step D1 is a step in which decimal visual acuity values have an equal interval. For example, the decimal step may be a step in which at least a part of the visual acuity values of the targets is increased by 0.1. The decimal step may be any step as long as the visual acuity value is linearly increased, and the interval is not limited to 0.1.

On the other hand, for example, a logarithmic step L1 is a step in which variations in the visual acuity values of the targets are provided at an equal interval in units of exponents (or power exponents). In other words, the logarithmic step L1 is a step in which the logarithmic visual acuity values have an equal interval. The logarithmic step can also be considered as a step in which the visual acuity value of the decimal display, which is calculated as the reciprocal of the visual angle of the target, is converted into base 10 logarithm and the logarithm is changed at an equal interval. For example, the logarithmic step may be a step in which at least a part of the visual acuity values of the targets is increased by 0.1 Log (common logarithm). The logarithmic step may be any step as long as the visual acuity value is logarithmically increased, and the interval is not limited to 0.1.

In the present example, steps that can be represented in the display format of the visual acuity value in the examination target are associated with the display format in advance. Therefore, the step of the visual acuity value is automatically selected based on the display format of the visual acuity value selected by the examiner. For example, in a case where the examiner selects the decimal button 71a for representing the visual acuity value as a decimal number, the control unit 20 automatically sets the change in the visual acuity value to the decimal step D1. In addition, based on the setting of the step of the visual acuity value, the control unit 20 cause the LCD 41 to display a visual acuity value group 75 represented in the step on the visual acuity value display setting screen 7 (refer to the example (b) of FIG. 4). For example, in the examination of the subject eye, the target is displayed on the LCD 3 with a size corresponding to the visual acuity value designated from the visual acuity value group.

In the present example, a configuration in which the step of the visual acuity value can be selected regardless of the display format of the visual acuity value in the examination target, may be applied. For example, in this case, the visual acuity value display setting screen 7 may include a step change button 73 for switching between the decimal step D1 and the logarithmic step L1 of the visual acuity value. For example, in a case where the examiner operates the LCD 41 to select the step change button 73 and switches the visual acuity value from the decimal step D1 to the logarithmic step L1, the control unit 20 cause the visual acuity value display setting screen 7 to display the visual acuity value group represented in the logarithmic step L1.

In addition, in the present example, a visual acuity value not included in the visual acuity value group based on the step of the visual acuity value in the examination target may be added. For example, in this case, the visual acuity value display setting screen 7 may include an input button 74 for the examiner to input any visual acuity value. For example, in a case where the examiner operates the LCD 41 to select the input button 74 and inputs any visual acuity value, the control unit 20 adds the input visual acuity value to the visual acuity value group. As an example, a visual acuity value of 1.8 which is not included in the visual acuity value group of the decimal step D1 may be newly added to the visual acuity value group.

### <Start of Visual Acuity Examination>

In a case where the examiner completes various settings of the examination target on the setting screen (type setting screen 6 and visual acuity value setting screen 7) of the LCD 41, the examiner starts the visual acuity examination for the subject eye. The examiner operates the switch unit 40 of the controller 4 to call up the examination screen for designating the target to be displayed on the display screen of the LCD 3and to be presented to the subject eye. The control unit 20 causes the LCD 41 to display the examination screen in response to the operation signal from the switch unit 40.

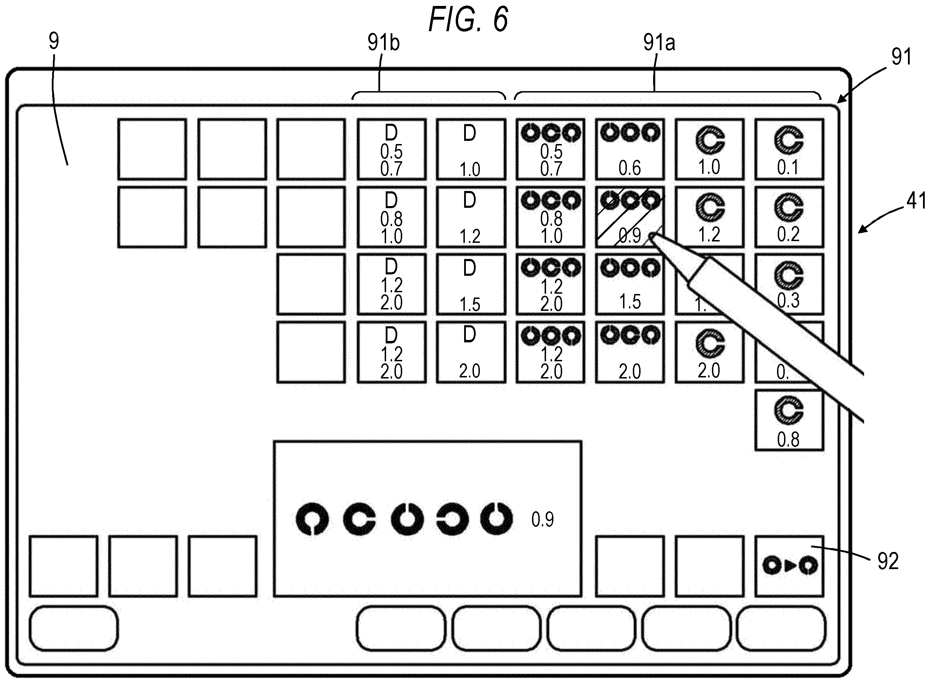

FIG. 6 illustrates an example of the examination screen 9. The examination screen 9 includes a target designation button 91 for designating the target to be displayed on the LCD 3. For example, the control unit 20 changes a configuration of the target designation button 91 and causes the LCD 41 to display the target designation button 91, based on the settings of displaying and non-displaying of the examination target from the type display change button 61, the settings of the display format of the visual acuity value from the format change button 71, the settings of the step of the visual acuity value, and the like. In the present example, with regard to the Landolt ring target and the alphabet target, the visual acuity value increased in the decimal step is set to be represented in the decimal display. Therefore, for example, a designation button 91a of the Landolt ring target is represented as the visual acuity value of the decimal display set from the visual acuity value group of the decimal step. Similarly, for example, a designation button 91b of the alphabet target is also represented as the visual acuity value of the decimal display set from the visual acuity value group of the decimal step.

The examiner operates the LCD 41 to designate the type of the target and the visual acuity value to be presented to the subject eye from the designation button 91a or the designation button 91b. The control unit 20 acquires a display size for displaying the corresponding target on the LCD 3, based on the operation signal from the LCD 41. For example, the control unit 20 may acquire the display size of the corresponding target by using a correspondence table in which the visual acuity value of the examination target and the display size are associated with each other.

For example, in the present example, the examiner designates the horizontal single-row target having the visual acuity value of 0.9 from the designation button 91a. The control unit 20 acquires the display size of the visual acuity value of 0.9, based on the correspondence table. The control unit 20 enlarges or reduces a reference image of the Landolt ring target stored in the memory 21, and randomly disposes the reference image, and thus, the control unit 20 causes the LCD 3 to display the horizontal single-row target having the display size corresponding to the visual acuity value of 0.9. The LCD 41 may display the examination target and the visual acuity value which are the same as those designated by the examiner.

The examiner asks the examinee the cut direction of the Landolt ring target displayed on the LCD 3, and switches at least either the cut direction of the Landolt ring target or the visual acuity value, according to an answer of the examinee. For example, the examiner may select a direction switching button 92 regardless of whether the answer is correct or incorrect, and may switch only the cut direction without switching the visual acuity value of the Landolt ring target. In addition, for example, in a case where the answer is correct, the examiner may select the designation button 91ato switch the visual acuity value of the Landolt ring target in the horizontal single-row target to a one stage higher visual acuity value. That is, a cut of the visual acuity value of the Landolt ring target may be increased, and the visual acuity value of the Landolt ring target may be switched to a value greater than a current value. As an example, the visual acuity value of the Landolt ring target may be switched from 0.9 to 1.0. In addition, for example, in a case where the answer is incorrect, the examiner may select the designation button 91ato switch the visual acuity value of the Landolt ring target in the horizontal single-row target to a one stage lower visual acuity value. That is, a cut of the visual acuity value of the Landolt ring target may be decreased, and the visual acuity value of the Landolt ring target may be switched to a value smaller than the current value. As an example, the visual acuity value of the Landolt ring target may be switched from 0.9 to 0.8.

### <Random Generation of Target>

Each time the control unit 20 obtains the operation signal from the designation button 91a or the direction switching button 92, the control unit 20 generates the horizontal single-row target and causes the LCD 3 to display the horizontal single-row target. That is, each time the control unit 20 obtains the operation signal, the control unit 20 causes the LCD 3 to display the horizontal single-row target in which the Landolt ring targets are randomly disposed. In the present example, in a case where the orders of alignment (that is, alignment of the columns and the rows) of the Landolt ring targets are identical to each other in the current horizontal single-row target and the subsequently switched horizontal single-row target, and the display positions of the Landolt ring targets overlap each other, the control unit 20 generates the horizontal single-row target so that the cut directions of the Landolt ring targets have different directions.

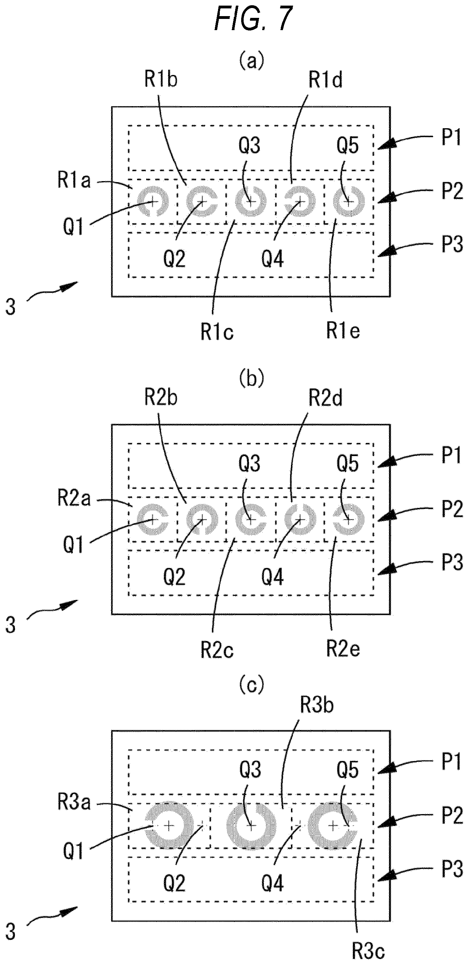

FIG. 7 illustrates examples of the current horizontal single-row target and the subsequent horizontal single-row target, and an example (a) of FIG. 7 illustrates the current horizontal single-row target. In the horizontal single-row target shown in the example (a) of FIG. 7, the Landolt ring targets having an identical visual acuity value are disposed in the order of one row and five columns at the middle stage position P2 among the upper stage position PI, the middle stage position P2, and the lower stage position P3 in the LCD 3. For example, a first row R1a to a fifth row R1e of the Landolt ring targets are aligned at an equal interval, and centers of Landolt rings are disposed to be located at respective display positions Q1 to Q5 on the screen. For example, the display position Q3 of the third row R1c of the Landolt ring targets is the center of the screen. Further, an example (b) of FIG. 7 and an example (c) of FIG. 7 illustrate the subsequent horizontal single-row target, the example (b) of FIG. 7 illustrates a state where the orders of alignment and the display positions of the Landolt ring targets are identical to the current horizontal single-row target, and the example (c) of FIG. 7 illustrates a state where the orders of alignment and some display positions of the Landolt ring targets are different from the current horizontal single-row target.

First, a case where the horizontal single-row target shown in the example (a) of FIG. 7 is switched to the horizontal single-row target shown in the example (b) of FIG. 7 will be described. Here, a case where the control unit 20 obtains the operation signal from the direction switching button 92 and changes the cut direction of the Landolt ring target will be described as an example. In the horizontal single-row target, the Landolt ring targets are disposed at an interval to secure a standardized predetermined distance. In a case where the cut direction of the Landolt ring target is switched from the current horizontal single-row target, the visual acuity value of the Landolt ring target is not changed, and the display size is not changed. Therefore, the same number of the Landolt ring targets are disposed at the same display position in the subsequent target. That is, in the subsequent horizontal single-row target illustrated in the example (b) of FIG. 7, the first row R2a to the fifth row R2e of the Landolt ring targets are disposed to be located at the display positions Q1 to Q5.

Since the display positions of respective rows of the Landolt ring targets are all the same in the current horizontal single-row target and the subsequent horizontal single-row target, the control unit 20 determines the cut direction so that the cut directions of the Landolt ring targets do not overlap each other in each row. More specifically, in the Landolt ring target of the current horizontal single-row target, the cut direction of the first row R1a is the downward direction. Therefore, in the Landolt ring target of the subsequent horizontal single-row target, the cut direction of the first row R2a is set to a direction different from the downward direction (that is, any direction of the upward direction, the leftward direction, and rightward direction). Similarly, the cut directions of the second to fifth rows are set to the directions which are not used in the corresponding Landolt ring target of the current horizontal single-row target.

Subsequently, a case where the horizontal single-row target shown in the example (a) of FIG. 7 is switched to the horizontal single-row target shown in the example (c) of FIG. 7 will be described. For example, when the visual acuity value of the Landolt ring target is switched from the current horizontal single-row target, in a case where the standardized predetermined distance can be secured even when the display size of the Landolt ring target increases, the same number of the Landolt ring targets are disposed at the same display position in the subsequent target. However, for example, in a case where the display size of the Landolt ring target increases and the predetermined distance cannot be secured, the number of the Landolt ring targets decreases in the subsequent target, and at least some Landolt ring targets are disposed at different display positions. Here, the following case will be described as an example. The control unit 20 obtains the operation signal from the designation button 91a, and changes the visual acuity value of the Landolt ring target so that the number of the Landolt ring targets decreases to three.

For example, in the subsequent horizontal single-row target illustrated in the example (c) of FIG. 7, the first row R3a and the third row R3c of the Landolt ring target are disposed at different display positions from those of the current horizontal single-row target illustrated in the example (a) of FIG. 7, and the second row R3b is disposed at the same display position Q3. In this case, the orders of alignment (alignment of the columns and the rows) of the Landolt ring targets are different from each other in the current horizontal single-row target and the subsequent horizontal single-row target. Therefore, the control unit 20 sets the cut directions of each of the first row R3a to the third row R3c to any direction of the upward direction, the downward direction, the leftward direction, and the rightward direction. That is, the control unit 20 sets the cut direction of the Landolt ring target in the subsequent horizontal single-row target, regardless of the direction of the Landolt ring target in the current horizontal single-row target. For example, the third row R1c of the Landolt ring target in the current horizontal single-row target and the second row R3b of the Landolt ring target in the subsequent horizontal single-row target are disposed at the same display position Q3. However, the cut directions of the Landolt ring targets may overlap each other. As a matter of course, since the third row R1c of the Landolt ring target in the current horizontal single-row target and the second row R3b of the Landolt ring target in the subsequent horizontal single-row target are disposed at the same display position Q3, the cut directions may be different from each other.

As described above, in a case where the number of the Landolt ring targets aligned in one row is four or more, the control unit 20 randomly generates the horizontal single-row targets so that all of the cut directions appear, namely the upward direction, the downward direction, the leftward direction, and the rightward direction. In addition, the horizontal single-row target may be randomly generated so that at least the Landolt ring targets adjacent to each other have different cut directions.

For example, in a case where the examiner switches the visual acuity value or the cut direction of the Landolt ring target presented to the subject eye, the control unit 20 generates the horizontal single-row target as described above, and controls the display of the LCD 3 to display the horizontal single-row target.

As described above, for example, the display format of the visual acuity value of the examination target used in the examination of the subject eye is set based on the operation signal, and the target to be presented to the subject eye is output based on the visual acuity value represented in the set display format. For example, in the related art, since it is difficult to freely select the display format of the visual acuity value of the examination target, the visual acuity value may not be represented in the display format desired by the examiner. Consequently, there is an aspect lacking of convenience in the related art. However, since the device has above-described configuration, the convenience is improved. The examiner can easily confirm the visual acuity value, and can promote the examination. In addition, for example, the examiner can change the display format of the visual acuity value of the target in accordance with the nationality of the examinee, and can cope with various situations.

In addition, for example, in the target presentation device of the present example, the examination target used in the examination of the subject eye includes the plurality of targets having different visual acuity values, and a variation in the visual acuity values of the plurality of targets is set in a predetermined step, based on the set display format of the visual acuity value. For example, in a case where the display format of the visual acuity value of the examination target is changed, the steps represented in the display format may also be changed. The display format of the visual acuity value is associated with the step in advance. In this manner, it is possible to easily determine the visual acuity value group of the target which can be presented to the subject eye.

In addition, for example, the target presentation device of the present example changes the predetermined step corresponding to the display format of the visual acuity value of the examination target to a different step, based on the operation signal. For example, in some cases, a plurality of steps (for example, the decimal step and the logarithmic step) represented in the display format may exist in the display format of the visual acuity value of the examination target. Since the initially set step according to the display format of the visual acuity value can be changed to another step, the visual acuity value group of the target which can be presented to the subject eye can be changed when needed.

In addition, for example, the target presentation device of the present example adds the target having a new visual acuity value represented in the display format of the visual acuity value of the examination target, based on the operation signal. For example, in this manner, the visual acuity value required by the examiner can be added to the visual acuity value group based on the variation (step) in the visual acuity values of the plurality of targets. In addition, it is possible to newly prepare the visual acuity value group including only the visual acuity values required by the examiner. The examiner can efficiently proceed with the examination by using the visual acuity value group described above.

In addition, for example, in the target presentation device of the present example, the type of the examination target is set based on the operation signal. For example, since the target of the examination target can be changed, the examination can be proceeded by using the type of the target familiar to the country or the region.

In addition, for example, the target presentation device of the present example acquires the display size of the target based on the visual acuity value input from the visual acuity value setting screen corresponding to the display format of the visual acuity value of the examination target, and causes the LCD 3 to display the target with the display size. For example, since the visual acuity value setting screen is switched for each display format of the visual acuity value of the examination target, the examiner can easily perform or change various settings.

In addition, for example, in a case where the target presentation device of the present example randomly aligns the plurality of targets in a straight line in at least one of the column direction and the row direction to generate the first target group displayed on the same screen of the LCD 3, the target presentation device sets the targets adjacent to each other to have different contents. In this manner, for example, even in a case where the alignment of the target groups is not determined in advance, it is possible to avoid appearance of the alignment of the targets which are not suitable for the examination, such as a case where the cut directions of the Landolt ring targets are all the same. Therefore, examination accuracy can be improved. In addition, in a case of a configuration in which the examiner can add any visual acuity value, since the alignment of the target groups cannot be determined in advance for each visual acuity value, above-described configuration is particularly effective.

In addition, for example, when the target presentation device of the present example randomly aligns the plurality of targets in a straight line in at least one of the column direction and the row direction to generate the second target group, that is switched from the first target group of the LCD 3 and displayed on the same screen of the LCD 3, , in a case where the order of alignment of the plurality of targets forming the second target group and the order of alignment of the plurality of targets forming the first target group are the same as each other, and the display position of the target in the second target group overlaps the display position of the target in the first target group, the target presentation device sets the targets to have different contents. That is, for example, when the current first target group is switched to the subsequent second target group, in a case where the display positions of the targets on the LCD 3 overlap each other, the target presentation device sets the subsequent target to have a different content. Therefore, the subject is less likely to remember the targets, and examination accuracy can be improved.

In addition, for example, when the target is the target having an orientation and the current first target group is switched to the subsequent second target group, in a case where the display positions of the targets on the LCD 3 overlap each other, the target presentation device of the present example sets the subsequent target to have a different orientation. In this manner, the subject is less likely to remember the direction of the target, and examination accuracy can be improved. In addition, since the targets have different orientations, the examination considering astigmatism can be proceeded as described later.

In addition, for example, when the target is the character target or the numerical symbol target and the current first target group is switched to the subsequent second target group, in a case where the display positions of the targets on the LCD 3 overlap each other, the target presentation device of the present example sets the subsequent target to have a different character or a different numerical symbol. In this manner, the subject is less likely to remember the character and the numerical symbol of the target, and examination accuracy can be improved.

### <Modified Example>

In the present example, a configuration in which a predetermined step is associated with the display format of the visual acuity value of the examination target as the initial setting has been described as an example. However, the present invention is not limited thereto. In the present example, a configuration in which the examiner can select any step without associating the display format of the visual acuity value of the examination target and the step with each other, may be applied. That is, a configuration in which any step which can be represented in a predetermined display format can be selected, regardless of the display format of the visual acuity value of the examination target, may be applied. In this manner, the examiner can freely set the visual acuity value group of the target which can be presented to the subject eye at each time in accordance with an examination purpose or content.

In the present example, a configuration in which either the decimal step D1 or the logarithmic step L1 is selected for the visual acuity values of the plurality of targets has been described as an example. However, the present invention is not limited thereto. In the present example, a configuration in which the logarithmic step can be set in more detail, may be applied. For example, the logarithmic step L1 is often used overseas for the visual acuity values of the plurality of targets. However, in some cases, the visual acuity values specified to the country or the region may be included, and the intervals may not be equal. Therefore, the logarithmic step D1 may be subdivided into steps in which the specified visual acuity value is added based on the logarithmic step.

In the present example, a configuration in which all of the Landolt ring targets of the visual acuity value group based on the predetermined step of the visual acuity value of the examination target are selected to be displayed on the LCD 3, has been described as an example. However, the present invention is not limited thereto. In the present example, a configuration in which it is possible to select the Landolt ring target, having individual visual acuity values forming the visual acuity value group, to be displayed on the LCD 3. For example, in this case, the visual acuity value display setting screen 7 may include a button (for example, a check box) for changing displaying and non-displaying of the individual visual acuity values. The control unit 20 may select only the Landolt ring target, having the visual acuity value, set as displaying out of the individual visual acuity values, to be displayed on the LCD 3.

In the present example, a configuration in which the fixed pattern is provided for the character target and the numerical symbol target has been described as an example. However, the present invention is not limited thereto. For example, in the character target and the numerical symbol target, displaying and non-displaying of the targets can be switched by one character. However, in this case, particularly in a case where the horizontal single-row target is generated, there is a possibility that the order of alignment of the character target may become an alignment which are not suitable for the examination of the subject eye. Therefore, the control unit 20 may generate the horizontal single-row target to avoid the alignment of the characters stored in the memory 21 in advance. As a matter of course, the examiner may set the alignment of the characters to be avoided, from the controller 4.

In the present example, a configuration in which the examiner operates the controller 4 to manually set at least one of the display format of the visual acuity value of the examination target, the step and the display target of the visual acuity value, addition of the visual acuity value, the type of the examination target, the alignment of the character target and the numerical symbol target, and the like, has been described as an example. However, the present invention is not limited thereto. For example, a configuration in which various settings are read and automatically set by using an SD card which can be attached to and detached from the device, or a cloud which can communicate with the device, may be applied.

In the present example, a configuration in which the display size of the Landolt ring target on the LCD 3 is acquired by using the correspondence table in which the visual acuity value of the examination target and the display size are associated with each other, has been described as an example. However, the present invention is not limited thereto. For example, a configuration in which the display size of the Landolt ring target is obtained by using a calculation expression for calculating the display size based on the visual acuity value of the examination target, may be applied. As a matter of course, the correspondence table and the calculation expression may be used in combination. In particular, in a case where the examiner can add a new visual acuity value, the visual acuity value and the display size which can be added are less likely to be prepared as the correspondence table in advance. Therefore, the display size may be acquired from the correspondence table for the visual acuity value group based on the predetermined step of the visual acuity value, and the display size may be acquired from the calculation expression for the newly added visual acuity value.

In the present example, a case where the horizontal single-row target is switched in the visual acuity examination has been described as an example. However, the present invention is not limited thereto. For example, it is conceivable that the same applies to a vertical single-row target having the plurality of Landolt ring targets and five Landolt ring targets disposed in a vertical single row (in other words, alignment of five rows and one column), or the character crowding target.

In the present example, a configuration in which the number of the targets is reduced in a case where the standardized predetermined distance cannot be secured, has been described as an example. However, the present invention is not limited thereto. For example, a configuration to change the display position of the target so that the predetermined distance can be secured without reducing the number of the targets, may be applied.

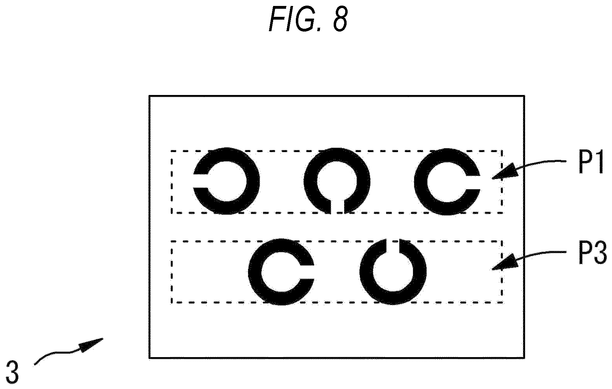

FIG. 8 illustrates a modified example of the target. For example, the visual acuity value of the Landolt ring target may be changed from the horizontal single-row target illustrated in the example (a) of FIG. 7 to a smaller value (that is, to have a larger display size). In this manner, in a case where the predetermined distance between the Landolt ring targets cannot be secured in the horizontal single row, the control unit 20 may divide the Landolt ring target into two stages, and may display the two stages on the LCD 3 as illustrated in FIG. 8. For example, the Landolt ring targets may be disposed at the upper stage position P1 in the order of one row and three columns, and the Landolt ring targets may be disposed at the lower stage position P3 in the order of one row and two columns.

Even in this case, the control unit 20 may set the cut directions of the Landolt ring targets adjacent to each other to have different directions. Furthermore, when the target in FIG. 8 is switched to the subsequent target, in a case where the display positions of the Landolt ring targets overlap each other, the cut directions of the Landolt ring targets may be set to have different directions.

In the present example, a configuration in which, with regard to one row in the horizontal single-row target, the Landolt ring targets are randomly disposed so that all of the cut directions appear in accordance with the number of the targets, and so that at least the targets adjacent to each other have different directions, has been described as an example. However, the present invention is not limited thereto. In the present example, a configuration in which the Landolt ring targets are randomly disposed considering a possibility that the subject eye is an astigmatic eye, may be further applied. In this case, in a case where an even number of the Landolt ring targets are aligned in one row, the control unit 20 may set the number of the vertical directions (upward direction and downward direction) and the number of the horizontal directions (leftward direction and rightward direction) of the cut directions to be the same as each other. In a case where an odd number of the Landolt ring targets are aligned in one row, the control unit 20 may set one of the number of the vertical directions and the number of the horizontal directions of the cut directions to be n, and may set the other to be n + 1. In a case where the horizontal single-row target is switched, a ratio between the number of the vertical directions and the number of the horizontal directions of the cut directions of the Landolt ring targets may be reversed. For example, considering the cut direction of the Landolt ring target as described above, the vertical direction and the horizontal direction of the cut direction can be evenly presented. Therefore, examination accuracy can be improved.

In the present example, a configuration in which the target presentation device 1 includes the housing 2 and the controller 4 has been described as an example. However, the present invention is not limited thereto. The target presentation device 1 may be used as a part of a subjective optometry device. That is, the target presentation device 1 may be used as a target presentation unit in the subjective optometry device. For example, the subjective optometry device may include a calibration optical system for calibrating the subject eye, and may subjectively measure optical characteristics of the subject eye. In this case, even in a case where the control unit 20 acquires the operation signal for changing a calibration degree to calibrate the subject eye in addition to the operation signal for changing the cut direction of the Landolt ring target or the operation signal for changing the visual acuity value of the Landolt ring target from the controller 4, the control unit 20 may randomly dispose the Landolt ring target by switching the horizontal single-row target. As a matter of course, the horizontal single-row target may be switched in accordance with a proceeding of a self-optometry program, and the Landolt ring target may be randomly disposed.

## Claims

1. A target presentation device for presenting a target to a subject eye, the target presentation device comprising:
a format setting means that sets a display format of a visual acuity value of an examination target used in an examination of the subject eye, based on an operation signal; and
an output means that outputs a target to be presented to the subject eye, based on the visual acuity value represented in the display format set by the format setting means.

2. The target presentation device according to claim 1,
wherein the examination target used in the examination of the subject eye includes a plurality of targets having different visual acuity values, and
the target presentation device further comprises a step setting means that sets the visual acuity values of the plurality of targets to have a predetermined step, based on the display format.

3. The target presentation device according to claim 2, further comprising:
a step changing means that changes the predetermined step to a step different from the predetermined step, based on an operation signal.

4. The target presentation device according to claim 1,
wherein the examination target used in the examination of the subject eye includes a plurality of targets having different visual acuity values, and
the target presentation device further comprises a step selection means that sets the visual acuity values of the plurality of targets to have any step, based on an operation signal, the visual acuity values being represented in the display format.

5. The target presentation device according to any one of claims 1 to 4, further comprising:
a target adding means that adds a target having a new visual acuity value represented in the display format, based on an operation signal.

6. The target presentation device according to any one of claims 1 to 5, further comprising:
a type setting means that sets a type of the examination target, based on an operation signal.

7. The target presentation device according to any one of claims 1 to 6,
wherein the output means is a target display means that displays the target,
the target presentation device further comprises a display control means that causes the target display means to display the target, and
the display control means acquires a display size in which the target is displayed on the target display means to cause the target display means to display the target in the acquired display size, based on the visual acuity value input from a visual acuity value setting screen corresponding to the display format.

8. The target presentation device according to claim 7, further comprising:
a generation means that randomly aligns a plurality of targets in a straight line in at least one of a column direction and a row direction to generate a first target group displayed on an identical screen of the target display means,
wherein the display control means causes the target display means to display the first target group generated by the generation means, and
the generation means randomly aligns the plurality of targets to set the targets adjacent to each other to have different contents.

9. The target presentation device according to claim 8,
wherein the first target group includes the plurality of targets having an identical first visual acuity value.

10. The target presentation device according to claim 8 or 9, further comprising:
a signal acquisition means that acquires a switching signal for switching the first target group displayed on the target display means,
wherein the generation means randomly aligns the plurality of targets in a straight line in at least one of a column direction and a row direction to generate a second target group displayed on the identical screen of the target display means, based on the switching signal from the signal acquisition means,
the display control means switches a display of the target display means from the first target group to the second target group, and
the generation means sets the targets to have different contents, in a case where an order of alignment of the plurality of targets forming the second target group is identical to an order of alignment of the plurality of targets forming the first target group, and a display position of the target in the second target group overlaps a display position of the target in the first target group.

11. The target presentation device according to claim 10,
wherein the second target group includes at least one of the plurality of targets having the identical first visual acuity value and the plurality of targets having a second visual acuity value different from the identical first visual acuity value.

12. The target presentation device according to any one of claims 8 to 11,
wherein the target is a target having an orientation, and
the generation means sets the targets to have different orientations.

13. The target presentation device according to any one of claims 8 to 11,
wherein the target is a character target or a numerical symbol target, and
the generation means sets the targets to have different characters or different numerical symbols.

14. A target presentation program used in a target presentation device for presenting a target to a subject eye,
wherein the target presentation program comprises instructions which, when the program is executed by a processor of the target presentation device, cause the target presentation device to perform:
a format setting step of setting a display format of a visual acuity value of an examination target used in an examination of the subject eye, based on an operation signal; and
an output step of outputting a target to be presented to the subject eye, based on the visual acuity value represented in the display format set in the format setting step.

15. The target presentation program according to claim 14,
wherein the target presentation program further comprises instructions which, when the program is executed by the processor of the target presentation device, cause the target presentation device to perform:
a generation step of randomly aligning a plurality of targets in a straight line in at least one of a column direction and a row direction to generate a first target group displayed on an identical screen of target display means; and
a display control step of causing the target display means to display the first target group generated in the generation step, and
in the generation step, the plurality of targets are randomly aligned to set the targets adjacent to each other are set to have different contents.
